# EUROPEAN PATENT APPLICATION

(11) **EP 4 108 654 A1**
(43) Date of publication of application: **28.12.2022**
(21) Application number: 21181256.5
(22) Date of filing: 23.06.2021
(51) Int. Cl.: C07C 235/08

(54) **PRODUCTION OF PANTHENOL**

(71) Applicant: DSM IP Assets B.V., 6411 TE Heerlen (NL)
(72) Inventor: MEDLOCK, Jonathan Alan, 4303 Kaiseraugst (CH); HOUSTON, Peter Louis, 4303 Kaiseraugst (CH)
(74) Representative: Seibel-Thomsen, Nadja

(57) **Abstract**

The present invention relates to a process for the production of panthenol from pantothenic acid or pantothenate.

## Description

The present invention relates to a novel and inventive process for the production of panthenol from pantothenic acid or pantothenate.

Panthenol is an alcohol derivative of pantothenic acid or vitamin B5 which is commercially used in cosmetics. Conventional production of panthenol is through chemical synthesis by condensing 3-aminopropanol with pantolactone. Aminopropanol however is a highly unwanted chemical reagent which should be avoided, also because it remains as an impurity in the final product.

Thus, there is an ongoing need for an efficient and sustainable process for the preparation of panthenol on an industrial scale which does not involve the use of aminopropanol as a reagent. Furthermore, there is a strong demand for more biological production processes, whereby the use or generation of hazardous substances is reduced or eliminated, such as e.g. for establishing a carbon-neutral route wherein fermentatively produced pantothenic acid is converted into highly pure panthenol.

Surprisingly, we now found a way for the production of panthenol wherein the use of aminopropanol can be avoided, said process comprising the conversion of pantothenic acid, pantothenate or pantothenic acid esters, particularly fermentatively produced pantothenic acid, into highly pure panthenol. As said process can use biotechnologically produced compounds including but not limited to pantothenic acid, said route furthermore can satisfy the need for production of more eco-friendly and carbon neutral products.

Particularly, the present invention is related to a process for the production of a compound according to formula (II): 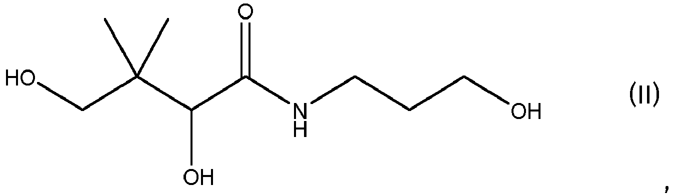 said process comprising the step of reduction of a compound according to formula (I) into a compound of formula (II) 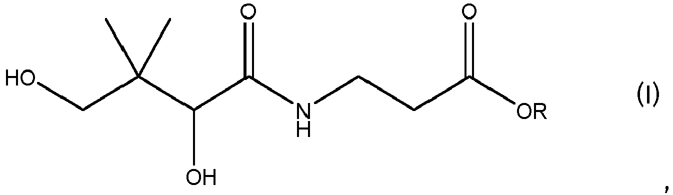 wherein R is selected from the group consisting of hydrogen, substituted or unsubstituted alkyl, or M, wherein a substituted or unsubstituted alkyl is preferably selected from a substituted or unsubstituted C1-C10 alkyl, such as e.g. C1, C2, C3, C4, C5, C6, C7, C8, C9 or C10-alkyl, more preferably selected from methyl, ethyl, n-propyl, iso-propyl, n-butyl, iso-butyl, tert-butyl, or benzyl; and wherein M is an alkali metal or earth alkali metal, particularly sodium, potassium, calcium, or magnesium.

It is well understood that in case of M being earth alkali metal, the respective ions are divalent and, hence, formally such metals in the formula (I) would be ½, e.g. ½ Ca, ½ Mg.

As used herein, the "compound of formula (II)" is also referred to as panthenol.

Suitable compounds according to formula (I) can be selected from pantothenic acid, respectively a salt thereof, such as e.g. calcium-pantothenate, sodium-pantothenate, magnesium-pantothenate, potassium-pantothenate or pantothenic acid esters, wherein those compounds can either be produced by a chemical or biocatalytic process or preferably via a fermentative process as e.g. described in WO0121772 or WO02057474.

The compounds according to formula (I) or (II) might be present in any configuration, such as e.g. in the (R) or (S)-configuration or occurring as racemate in (R/S)-configuration, whereby the (R) or (R/S)-configuration is preferred. Typically, if the compounds according to formula (I) are produced by a fermentation process, a percentage of at least about 95%, such as e.g. about 97, 98, 99 or event 100% of (R)-configuration based on total pantothenic compounds can be expected.

As used herein, such process combining chemical production steps and biotechnological steps is referred to as a "hybrid process".

As used herein, the term "pantothenic compounds" includes but is not limited to pantothenic acid, pantothenate, pantothenic acid ester and panthenol.

The reduction of a compound according to formula (I) to produce the compound according to formula (II) as described herein includes the use of reducing agents, particularly wherein the reducing agents are selected from the group consisting of lithium aluminium hydride (LiAlH₄), sodium borohydride (NaBH₄), potassium borohydride (KBH₄), and lithium borohydride (LiBH₄).

In one embodiment, said reduction as described herein leading to panthenol, with particularly at least about 95% panthenol based on total pantothenic compounds, is performed in the presence of an additive or activating agent, particularly wherein said agents and/or additives are selected from silanes such as chlorotrimethyl silane, iodine, Lewis acids such as zinc chloride or calcium chloride, or crown ethers.

Usually, such reduction processes as described herein are performed in the presence of a suitable solvent or a mixture of solvents.

In one embodiment, said reduction step as described herein is performed in the presence of a solvent, particularly wherein the solvent is a non-aqueous, organic, polar or non-polar solvent. Suitable solvents might be selected from alcohols and polyols, esters, ethers, amides, nitriles or hydrocarbons with or without substitutions, with a preference for substituted hydrocarbons. Particularly suitable solvents for the performance of the reduction step according to the present invention are selected from alcohols, ethers, amides and (substituted or unsubstituted) hydrocarbons.

The term "solvent" as used herein is understood as meaning a solvent which does not take part in a chemical reaction in the reaction medium and under the operating conditions, and which is inert to both the reactants and the reaction products. It is however not excluded by theory, that the solvent may form an intermediate solvate or additive with the reducing agent.

The reduction step leading to panthenol as of the present invention might be carried out at a suitable temperature, particularly at about 0 to about 100°C, preferably at a temperature of about 0 to about 40°C.

In one aspect, the present invention is directed to production of panthenol, preferably in (R)-configuration, wherein at least about 95%, such as e.g. about 97, 98, 99 or even 100% panthenol based on total pantothenic compounds is generated, preferably with at least about 95% present as (R)-panthenol based on total pantothenic compounds, wherein the formation of by-products, especially formation of aminopropanol, could be reduced or eliminated.

Particularly, the present invention is related to a product comprising panthenol in a purity of at least about 95%, preferably of at least about 97, 98, 99 or 100% as measurable via known methods including but not limited to HPLC or qNMR analysis, wherein the product is substantially free of impurities, such as e.g. aminopropanol, pantolactone and/or pantoic acid.

As used herein, the term "substantially free of" in the context of impurities means concentration (total amount) of aminopropanol, pantolactone and/ or pantoic acid of less than 0.1%, most preferably below any limit of detection as measured by any known method including but not limited to HPLC or qNMR. It furthermore refers to a product wherein aminopropanol, pantolactone and/or pantoic acid are not used (neither as intermediates nor as reagents) in the production process and thus, in contrast to the commonly used chemical process, no traceable amounts thereof are present, as it can be measured via known methods including but not limited to HPLC or qNMR.

The process as described herein comprising reduction of the compound according to formula (I) might also involve the use of a hydrogenation step, such as particularly wherein pantothenic acid, preferably bio-based pantothenic acid, is first converted into pantothenic acid ester and in a further step said pantothenic acid ester is then converted via a hydrogenation step into highly pure panthenol, particularly said second step being carried out in the presence of a transition metal complex and a gaseous mixture comprising at least about 10 to about 90% H₂ and wherein said process is not carried out in the presence of aminopropanol.

It is well understood, that the term bio-based as used herein refers to a product which is prepared by fermentation.

The esterification of pantothenic acid can be performed by standard means in the art, including but not limited to biocatalytic or fermentative processes. Chemical esterification, e.g. by reacting pantothenic acid with the respective alcohol in the presence of an acid is however preferred.

As used herein, the term "by-product" or "side-product" is used interchangeably herein and includes but is not limited to pantoic acid, pantolactone, aminopropanol or solvents present in the final product.

The product comprising the compound of formula (II) can be purified (when needed) using commonly known methods, such as e.g. distillation as described in e.g. US20120149903.

The invention is illustrated by the following Examples.

All percentages are related to the weight.

### Example 1: Reduction of methyl pantothenate with lithium aluminium hydride

A solution of lithium aluminium hydride (2.4M in THF, 0.44 ml) was diluted with THF (1 ml) and cooled to approximately 5°C. A solution of methyl pantothenate (0.2 g, 95% purity) in THF (0.7 ml) was added dropwise over 15 minutes and the reaction mixture was stirred for a further 1 hour at 5-10°C. The mixture was quenched with saturated sodium sulfate solution and the organic layer was separated, dried and evaporated to give 0.128 g of panthenol (94.8% purity by qNMR).

## Claims

1. A process for the production of a compound of formula (II): 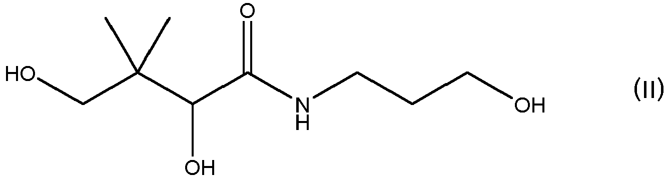 said process comprising the step of reduction of a compound according to formula (I) to the compound of formula (II) 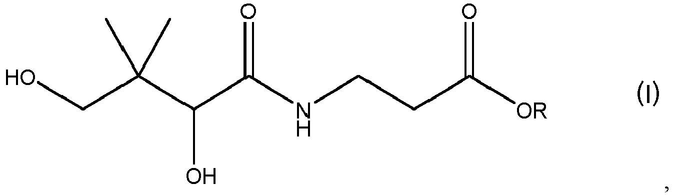 wherein R is selected from the group consisting of hydrogen, substituted or unsubstituted alkyl, and M, preferably wherein the substituted or unsubstituted alkyl is C1-C10 alkyl, such as e.g. C1, C2, C3, C4, C5, C6, C7, C8, C9 or C10-alkyl, more preferably selected from methyl, ethyl, n-propyl, iso-propyl, n-butyl, iso-butyl, tert-butyl, or benzyl; and wherein M is an alkali metal or earth alkali metal, particularly sodium, potassium, calcium, or magnesium.

2. The process according to claim 1, wherein M is selected from ½ calcium, ½ magnesium, sodium or potassium.

3. The process according to claim 1 or 2, wherein the compound according to formula (I) is selected from pantothenic acid, sodium-pantothenate, calcium-pantothenate, magnesium-pantothenate, or potassium-pantothenate, preferably wherein said compounds are fermentatively produced.

4. The process according to claim 3, further comprising the step of preparing said compounds of formula (I) by esterifying pantothenic acid or pantothenate with the respective alcohol.

5. The process according to any one of claims 1 to 4, wherein the percentage of panthenol is at least about 95% based on total pantothenic compounds.

6. The process according to any one of claims 1 to 5, wherein the reduction step is carried out in the presence of a reducing agents, preferably selected from the group consisting of of lithium aluminium hydride (LiAlH₄), sodium borohydride (NaBH₄), potassium borohydride (KBH₄), and lithium borohydride (LiBH₄).

7. The process according to any one of claims 1 to 6, wherein the reduction step is carried out in the presence of an additive or activating agent, preferably selected from silanes such as chlorotrimethyl silane, iodine, Lewis acids such as zinc chloride or calcium chloride, or crown ethers.

8. The process according to any one of claims 1 to 7, wherein the reduction step is carried out in the presence of a solvent selected from non-aqueous, organic, polar or non-polar solvents, particularly solvents selected from alcohols and polyols, esters, ethers, hydrocarbons with or without substitutions, amides or nitriles.

9. The process according to any one of claims 1 to 8, wherein the reduction step is carried out at a temperature of about 0 to about 100°C, preferably of about 0 to about 40°C.

10. The process for the production of a product comprising a compound of formula (II), said process comprising a reduction step according to any one of claims 1 to 9, wherein the product is substantially free of aminopropanol.

11. A product produced via the process according to claim 10 comprising a percentage of aminopropanol in the range of less than 0.1% as measured by HPLC or qNMR.
